# EUROPEAN PATENT SPECIFICATION

(11) **EP 4 536 325 B1**
(45) Date of publication and mention of the grant of the patent: **06.05.2026**
(21) Application number: 23728382.5
(22) Date of filing: 26.05.2023
(51) Int. Cl.: A61M 5/20

(54) **AN ENERGY HARVESTING SUBASSEMBLY OF A MEDICAMENT DELIVERY DEVICE**
EINE ENERGIE ERZEUGENDE UNTERANORDNUNG EINER MEDIKAMENTENABGABEVORRICHTUNG
SOUS-ENSEMBLE DE RECUPERATION DE L'ENERGIE D'UN DISPOSITIF D'ADMINISTRATION DE MÉDICAMENT

(30) Priority: 09.06.2022 EP 22178121
(43) Date of publication of application: 16.04.2025
(73) Proprietor: SHL Medical AG, 6302 Zug (CH)
(72) Inventor: SÄLL, Daniel, 131 28 Nacka Strand (SE)
(86) International application number: PCT/EP2023/064161
(87) International publication number: WO 2023/237356

(56) References cited:
- EP-A1- 3 326 671
- WO-A1-2016/055305

## Description

### TECHNICAL FIELD

The present disclosure generally relates to medicament delivery devices such as autoinjectors, and particularly concerns a subassembly adapted to provide digital confirmation of a completed injection.

### BACKGROUND

A number of medical conditions require injections. These days, a number of different injection devices exist, including various types of pen injectors, autoinjectors and on-body devices. Although many of these devices have enabled major improvements in the management of a number of medical conditions, various limitations do still exist in the current technology. Not least amongst these are the difficulties faced by patients that require frequent injections and by patients that need to inject particularly viscous drugs. In considering these problems, the applicant has appreciated that various developments could be made to help improve the medicament delivery devices on the market today. For example, the injection devices on the market are typically only used once before being disposed of which is not preferable from a sustainability point of view. Prior art injection devices are disclosed in WO 2016/055305 A1 and EP 3 326 671 A1.

### SUMMARY

An object of the present disclosure is to provide a subassembly for a medicament delivery device which solves, or at least mitigates problems of the prior art.

According to a first aspect of the present disclosure, there is provided a subassembly of a medicament delivery device, the subassembly comprising: a housing extending along a longitudinal axis and having a proximal end and distal end; a rear cap arranged inside the housing at the distal end, the rear cap comprising a flexible arm that is flexible in a radial direction perpendicular to the longitudinal axis, the flexible arm comprising a protrusion; a U-bracket arranged radially inside the rear cap and extending from a distal end of an inner space of the rear cap to a proximal end of the rear cap, the U-bracket comprising at least one distally facing surface on its proximal end that abuts against a respective proximally facing surface of the rear cap, and wherein a distal end of the U-bracket is spaced apart from the rear cap by a gap; a plunger rod arranged radially inside the U-bracket, the plunger rod comprising a proximally facing surface that abuts against a distally facing surface of the protrusion of the rear cap; a spring arranged radially inside the plunger rod, the spring is compressed by a distally facing surface of the plunger rod and a proximally facing surface of the U-bracket; a piezo-electric energy harvesting unit arranged distally with respect to the rear cap and comprising a first member being movable with respect to a second member for generating electrical energy, the first member being arranged adjacent the rear cap so that, when the spring is released, the spring force acts on the U-bracket to close the gap to cause the first member to move in relation to the second member; and a power management unit configured to harvest the electrical energy generated when the first member moves in relation to the second member and to provide the electrical energy to a data transmission device.

Embodiments of the present disclosure advantageously provide for harvesting electrical energy from the motion of the U-bracket as it impacts on the rear cap. When the U-bracket hits the rear cap, the impact force is transferred to the first member of the piezo-electric energy harvesting unit so that it moves with respect to the second member of the piezo-electric energy harvesting unit. This allows energy to be harvested by the piezo-electric energy harvesting unit which can be used for powering the data transmission device to send a digital confirmation of an injection to a receiver hub or an external receiver device. Such digital confirmation includes for example a time stamp and serial number of the injector.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the components thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially" refer to a circumference or a circumferential direction 301 relative to an axis 5, typically a central axis extending in the direction of the longest extension of the device and/or component. Similarly, "radial" or "radially" refer to a direction extending radially 302 relative to the axis, and "rotation", "rotational" and "rotationally" refer to rotation relative to the axis.

According to one embodiment, the piezo-electric energy harvesting unit may be arranged in a rear lid attached to the distal end of the housing. The rear lid is typically permanently fixed to the housing but may in some possible implementations be removable.

According to one embodiment, a diameter of the first member may be substantially equal to an inner diameter of the lid housing the first member and the second member. Generally, the larger the first member is, a higher power can be generated by the energy harvesting unit. Thus, increasing the diameter of the first member advantageously provides for a higher generated power.

According to one embodiment, the rear cap may comprise a pin extending from the distal end of the rear cap towards the first member. The pin hits the first member when the U-bracket hits the rear cap and enables a more efficient transfer of the motion from the U-bracket to the first member. Preferably, the pin is attached to a flexible portion of the rear cap. The flexible portion flexes as the U-bracket impact on the opposite side from the pin.

According to one embodiment, when the spring is released, the spring force acts on the U-bracket to close the gap and impact on a surface of the rear cap, whereby the pin is caused to impact on the first member. The surface and the pin are on opposite sides of the flexible portion of the rear cap.

According to one embodiment, the U-bracket may be configured to bounce on the surface of the rear cap multiple times. This produces multiple voltage bursts in the energy harvesting unit and results in a higher generated power compared to a single burst.

According to one embodiment, the at least one distally facing surface of the U-bracket may be comprised in a respective radially extending protrusion.

According to one embodiment, the subassembly may comprise a guide rod arranged radially inside the spring, the guide rod comprising a flange on its distal end that abuts against the distal end of the spring and a distal end of the U-bracket.

According to one embodiment, the at least one distally facing surface of the U-bracket may be inclined with respect to the longitudinal axis. The inclined surface(s) facilitates the radial compression of the U-bracket as it interacts with the proximal surface of the rear cap.

According to one embodiment, when the plunger rod is moved proximally in relation to the U-bracket the spring is released and decompressed, and when the plunger rod reaches to a position where the plunger rod is outside the U-bracket, the U-bracket is radially compressed so that the distally facing surface of the U-bracket is moved away from the proximally facing surface of the rear cap to be movable in the distal direction under the influence of a force from the spring.

According to one embodiment, the first member is a piezo electric membrane being movable with respect to the second member being a support for the piezo electric membrane.

According to one embodiment, when the spring is released, a click sound is generated by an impact of a distally facing surface of the U-bracket and a proximally facing surface of the rear cap. This advantageously provides a confirmation of the injection to the user.

According to one embodiment, the data transmission device may include a Bluetooth transmitter. Preferably, the Bluetooth transmitter is a Bluetooth low energy (BLE) transmitter.

There is further provided a medicament delivery device comprising the subassembly of any of the herein disclosed embodiments.

Generally, all terms used in the claims are to be interpreted according to their ordinary meaning in the technical field, unless explicitly defined otherwise herein. All references to "a/an/the member, apparatus, component, means, etc." are to be interpreted openly as referring to at least one instance of the member, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 is a perspective view of an autoinjector according to embodiments of the present disclosure;
Fig. 2 is an exploded view of the medicament delivery device according to embodiments of the present disclosure;
Fig. 3A and Fig. 3B illustrates two different views of a rear cap according to embodiments of the present disclosure;
Fig. 4 illustrates a U-bracket of the subassembly according to embodiments of the present disclosure;
Fig. 5 illustrates the plunger rod of the subassembly according to embodiments of the present disclosure;
Fig. 6A illustrates one view of a conceptual piezo-electric energy harvesting unit according to embodiments of the present disclosure;
Fig. 6B illustrates another view of the conceptual piezo-electric energy harvesting unit according to embodiments of the present disclosure;
Fig. 7 illustrates a guide rod of the subassembly according to embodiments of the present disclosure;
Fig. 8 is a cross-section of the subassembly according to embodiments of the present disclosure;
Fig. 9 is a cross-section of the subassembly according to embodiments of the present disclosure;
Fig. 10 is a cross-section of the subassembly when injection has started according to embodiments of the present disclosure;
Fig. 11 is a cross-section of the subassembly when the U-bracket has closed the gap according to embodiments of the present disclosure;
Fig. 12 illustrates a further possible implementation of the subassembly including inductive energy harvesting;
Fig. 13 illustrates a further possible implementation of the subassembly including inductive energy harvesting; and
Fig. 14 illustrates a further possible implementation of the subassembly employing piezoelectric energy harvesting.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like members throughout the description.

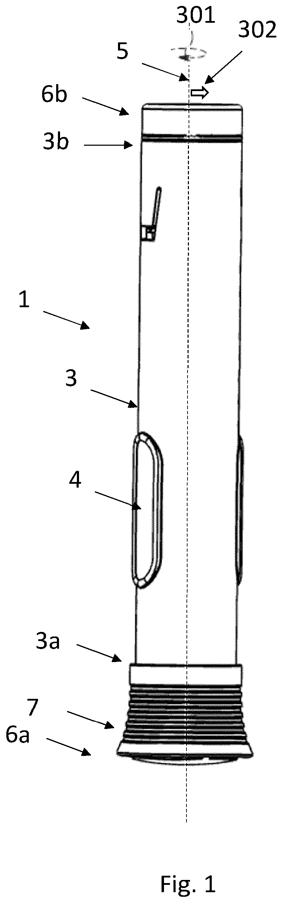

Fig 1 shows an example of a medicament delivery device 1 such as an autoinjector according to embodiments of the present disclosure. The medicament delivery device 1 is configured to expel medicament from a medicament container 11 via a medicament delivery member such as a needle, to a user at a dose delivery site. The medicament delivery device 1 extends from a proximal end 6a to a distal end 6b relative to the axis 5.

The medicament delivery device 1 comprises a housing 3 with a window 4. The housing 3 has a proximal end 3a and a distal end 3b. A front cap 7 covers the proximal end 6a of the housing 3.

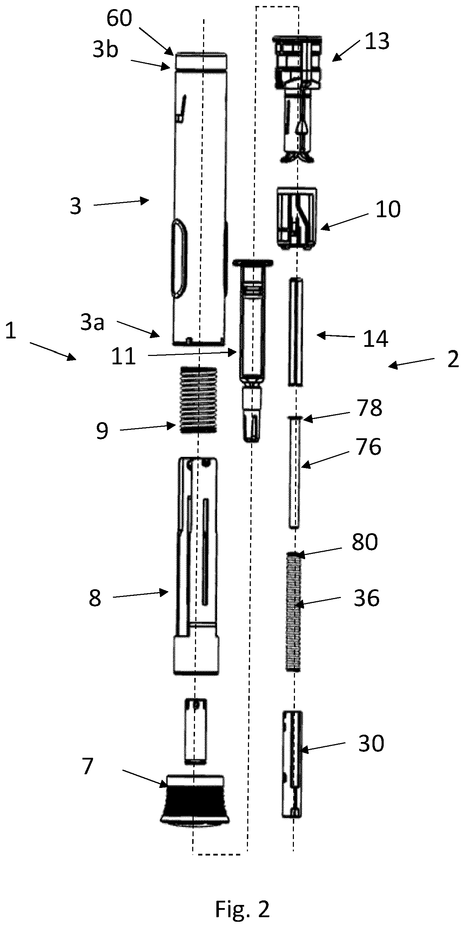

Fig. 2 is an exploded view of the medicament delivery device 1 which further comprises a cover structure 8 arranged in the housing 3 and extends proximally from the proximal end 3a. The cover structure 8 is configured to be moved linearly relative to the housing 3 from an extended position to a retracted position in which the cover structure 8 is received further in the housing 3 and in which a medicament delivery member such as a needle is exposed.

The cover structure 8 is biased in the proximal direction towards the extended position by a spring 9.

As is often the case, the medicament delivery device 1 comprises a rotator 10 configured to lock the cover structure 8 in its extended position after use to shield the needle from the user to prevent sharp injury.

The medicament delivery device 1 comprises the subassembly 2 which will now be described in more detail with reference to subsequent drawings. The subassembly 2 comprises a rear cap 13, a U-bracket 14, a plunger rod 30, a spring 36, and a piezo-electric energy harvesting unit arranged in the distal lid 60 of the housing 3. Optionally, the subassembly 2 comprises a guide rod 76.

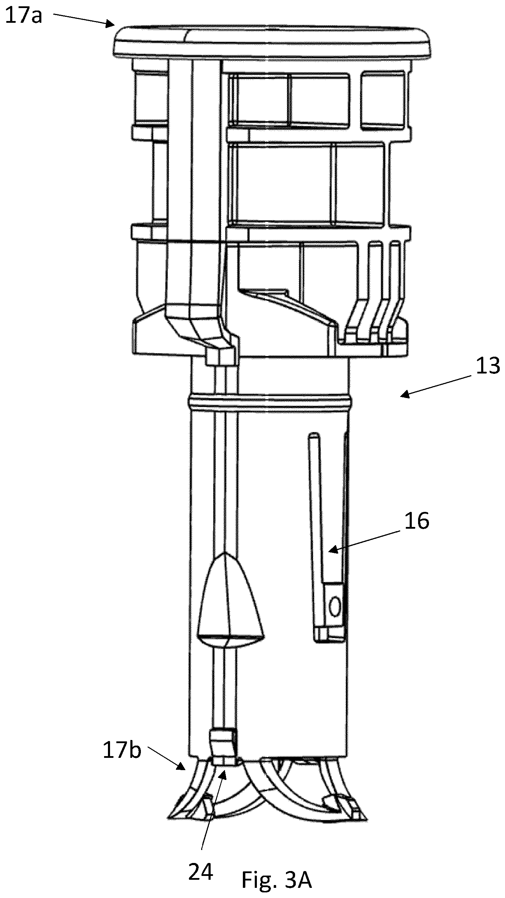

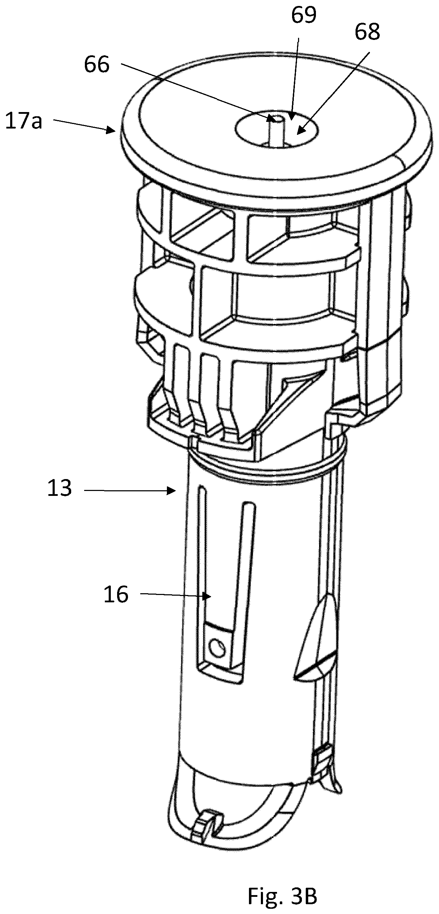

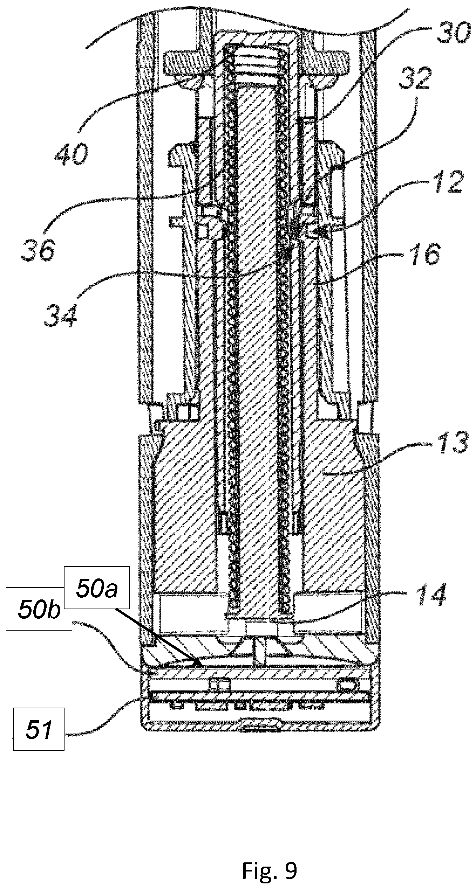

Fig. 3A and Fig. 3B illustrates two different views of the rear cap 13 that, in the subassembly 2, is arranged inside the housing 3 at the distal end 6b. The rear cap 13 comprising a flexible arm 16 that is flexible in a radial direction 302 perpendicular to the longitudinal axis 5. The flexible arm 16 comprises a protrusion 12 extending radially inwards and is better seen in fig. 9. The flexible arm 16 is made from a cut-out in the rear cap 13 and has a longitudinal extension parallel with the longitudinal axis 5.

Further, the rear cap 13 comprises a pin 66 extending from the distal end 17a of the rear cap 13 in a distal direction. The pin 66 is preferably attached to a flexible portion 68 of the rear cap 13 that allows for axial motion of the pin 66 along the longitudinal axis 5 when the flexible portion 68 flexes or moves along the longitudinal axis 5. The flexible portion 68 forms or defines a circular hole in the distal end 17a of the rear cap. The pin 66 extends out from the hole 69.

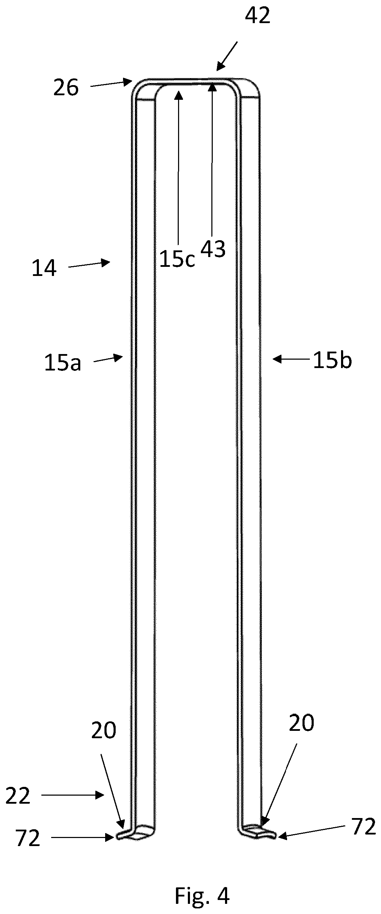

Fig. 4 illustrates a U-bracket 14 of the subassembly 2. The U-bracket 14 is generally U-shaped and comprises two parallel legs 15a, 15b interconnected by a distal connection bridge 15c. The connection bridge 15c defines the distal end 26 of the U-bracket. The U-bracket 14 comprises at least one distally facing surface 20 on its proximal end 22, here the distally facing surfaces 20 of the U-bracket 14 are comprised in a respective radially extending protrusion 72. In the subassembly 2, the distally facing surfaces 20 abuts against a respective proximally facing surface 24 of the rear cap 13.

The U-bracket 14 is flexible so that the two parallel legs 15a, 15b can flex towards and away from each other, so that they are no longer parallel. In its neutral state the legs 15a, 15b are substantially parallel.

The U-bracket 14 comprises a distally facing surface 42 on its distal end 26 that cooperates with a proximally facing surface 70 of the rear cap to generate a click-sound to confirm injection. Further, the U-bracket 14 comprises a proximally facing surface 43 on its distal end 26 that participates in compressing the spring 36, as will be discussed further below.

Further, the distally facing surfaces 20 of the U-bracket are inclined with respect to the longitudinal axis 5.

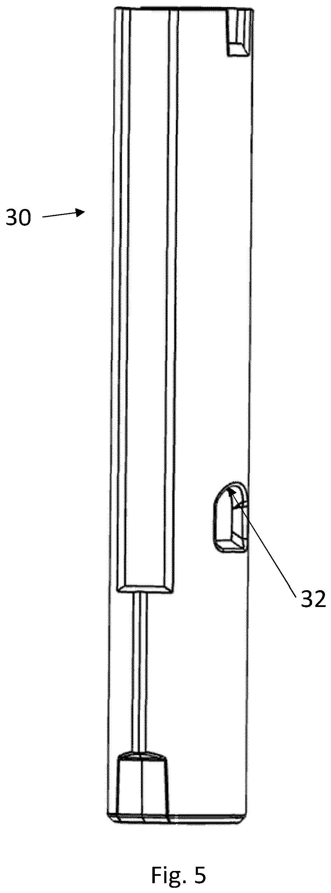

Fig. 5 illustrates the plunger rod 30 which comprises a proximally facing surface 32. In the subassembly 2, the plunger rod 30 is arranged radially inside the U-bracket 14. Further, the proximally facing surface 32 abuts against a distally facing surface 34 of a protrusion of the rear cap 13. The proximally facing surface 32 defines a groove, cut-out, or through-hole of the plunger rod 30.

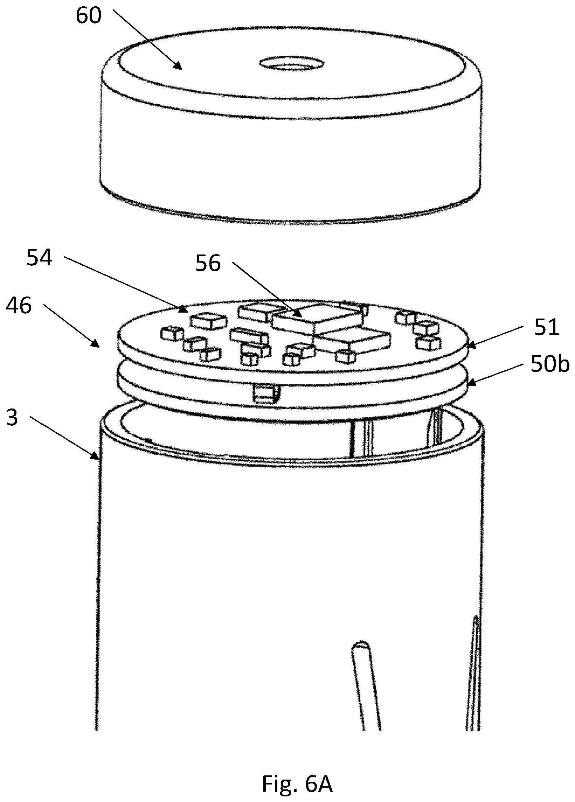

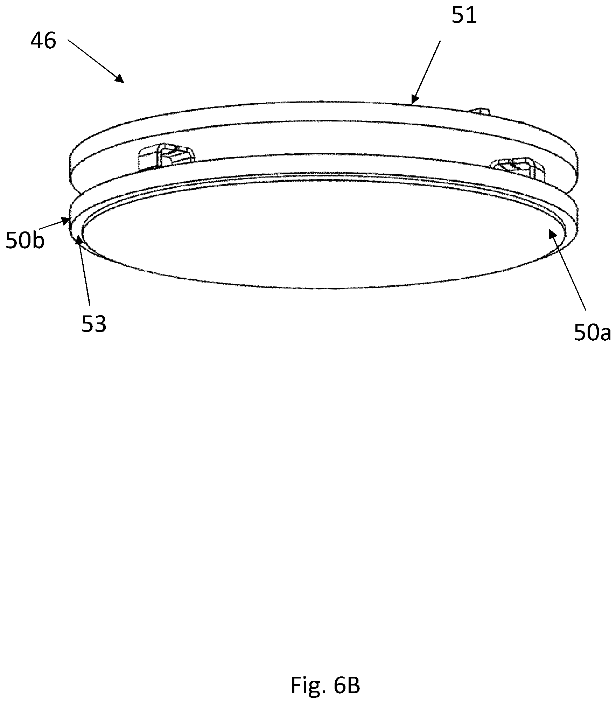

Figs. 6A-B illustrates two perspective views of a conceptual piezo-electric energy harvesting unit 46. In the subassembly, the piezo-electric energy harvesting unit 46 is arranged distally with respect to the rear cap 8 and comprises a first member 50a being movable with respect to a second member 50b. The motion of the first member 50a causes a generation of electrical energy due to the piezo-electric property of the first member 50a. In the subassembly 2, the first member 50a is arranged adjacent the rear cap 13 so that, when the spring 36 is released, the spring force acts on the U-bracket 14 to close a gap 28 between the U-bracket and the rear cap 13 to cause the first member 50a to move in relation to the second member 50b. Preferably, the piezo-electric energy harvesting unit 46 is arranged in a rear lid 60 attached to the distal end 3b of the housing 3.

The first member 50a is a piezo electric membrane attached to the proximal side 53 of the second member 50b which may serve as a support comprising electrodes for the piezo electric membrane. Further, to maximize the power generation, the diameter of the first member 50a is a large as possible, preferably substantially equal to an inner diameter of the lid 60 where the first member 50a and the second member 50b are arranged. The first member 50a and the support 51 are spaced apart. It is understood that piezoelectric energy harvesting units are *per* se known to the skilled person and their operation will not be discussed in detail herein.

Further, a conceptual power management unit 54 is shown in fig. 6A. The power management unit 54 configured to harvest the electrical energy generated when the first member 50a moves in relation to the second member 50b and to provide the electrical energy to a data transmission device 56. The data transmission device 56 and the power management unit 54 are supported on a PCB 51 arranged in the lid 60. The PCB 51 also carries other components needed to transmit a digital signal such as a processor and an antenna. The data transmission device 56 preferably includes a Bluetooth transmitter such as a Bluetooth low energy transmitter configured to transmit a digital confirmation to a hub or external receiver. The receiver hub or external device generates a time stamp of the digital confirmation. The digital confirmation comprises a serial number of the medicament delivery device 1 and/or of the medicament that was injected.

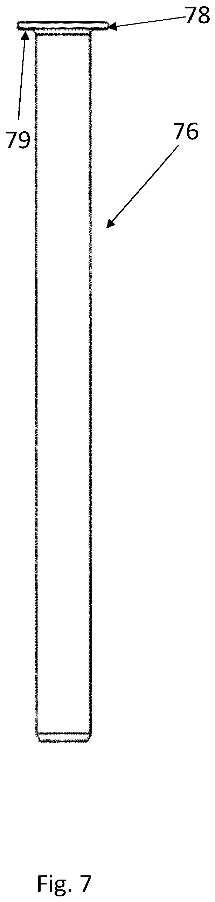

Fig. 7 illustrates a guide rod 76. In the subassembly 2, the guide rod 76 is arranged radially inside the spring 36 and the spring is movable in relation to the guide rod 76. The guide rod 76 comprises a flange 78 on its distal end that comprises a proximally facing surface 79 that abuts against the distal end 80 of the spring and a distal end of the U-bracket 14. The flange 78 extends radially from the guide rod body and abuts against the proximally facing surface 43 on the distal end 26 of the U-bracket 14.

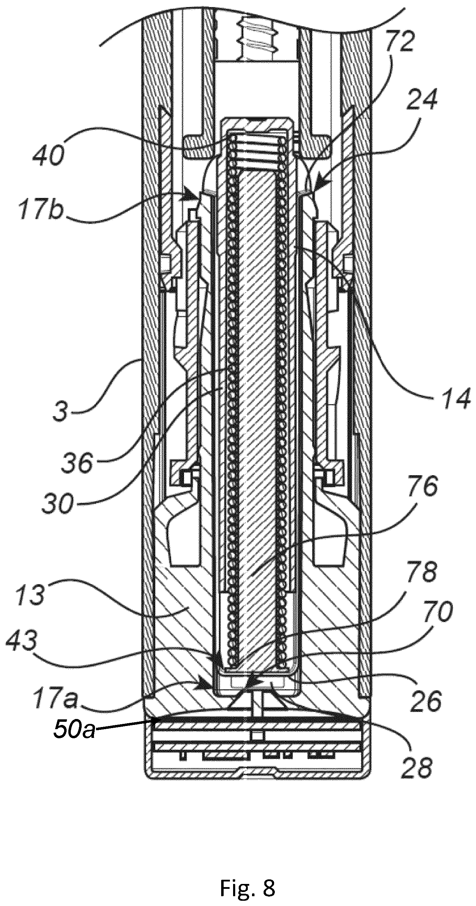

Fig. 8 and fig. 9 are cross-sections of the subassembly 2 prior to injection. The rear cap 13 is shown arranged radially inside the housing 3 and comprising the flexible arm 16 having a protrusion 12 better seen in fig. 9. The U-bracket 14 with the protrusions 72 is arranged radially inside the rear cap 13 from a distal end 17a to a proximal end 17b of the rear cap 13 inner space. The distally facing surface 20 of the protrusion 72 abuts against the proximally facing surface 24 of the rear cap 13. The proximally facing surface 24 is located at the proximal end 17b of the rear cap 13. The distal end 26 of the U-bracket 14 is spaced apart from the rear cap 13 by a gap 28.

The plunger rod 30 is arranged radially inside the U-bracket 14. The plunger rod 30 comprises a proximally facing surface 32 that abuts against the distally facing surface 34 of the protrusion 12 of the rear cap 13.

The spring 36 is arranged radially inside the plunger rod 30. The spring is compressed between a distally facing surface 40 of the plunger rod 30 proximal end and a proximally facing surface 43 of the U-bracket. The distal end of the spring 36 abuts against the flange 78 of the guide rod 76 that is arranged radially inside the spring 36.

In the state of the subassembly 2 shown in fig. 8 and fig. 9, the spring 36 is compressed. However, as is better seen in fig. 9, the plunger rod 30 is prevented from moving in the distal direction by the spring force due to the engagement between the distally facing surface 34 protrusion 12 of the rear cap and the proximally facing surface 32 of the plunger rod 30 as defined by the groove or through-hole discussed in relation to fig. 5. The U-bracket 14 is prevented from moving distally by the spring force with respect to the rear cap 13 by the engagement between the protrusion 72 and the proximal surface 24 of the rear cap 13. Since the plunger rod 30 and the U-bracket 14 are both prevented from moving longitudinally with respect to the rear cap 13, the spring 36 is compressed by the distally facing surface 40 of the plunger rod and the proximally facing surface 43 of the U-bracket 14. Further, the guide rod 76 which receives the spring 36 on its flange 78 is prevented from moving distally by its engagement with the proximally facing surface 43. Overall, a gap 28 is left between the U-bracket 14 and the rear cap 13 surface 70.

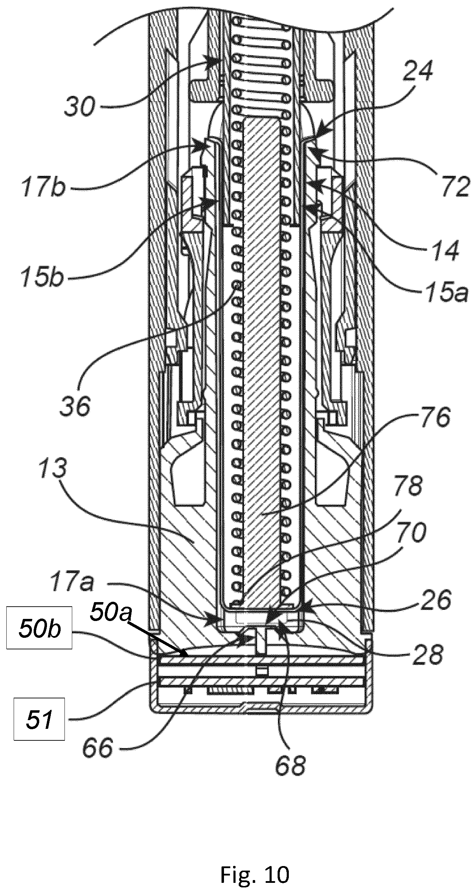

When injection is started, as illustrated in fig. 10, the plunger rod 30 moves inside the housing 3 in a proximal direction to cause expulsion of medicament from a medicament container 11. When the injection is initiated the protrusion 12 of the rear cap 13 are radially moved outwards so that the engagement between the proximally facing surface 32 of the plunger rod 30 and the distally facing surface 34 of the protrusion 12 is released. This enables the spring 36 to decompress and push the plunger rod 30 in the proximal direction. However, here in this initial stage of the injection, the plunger rod 30 is still located radially between the spring 36 and the legs 15a and 15b of the U-bracket 14. The legs 15a-b are therefore prevented by the plunger rod 30 from being compressed towards each other.

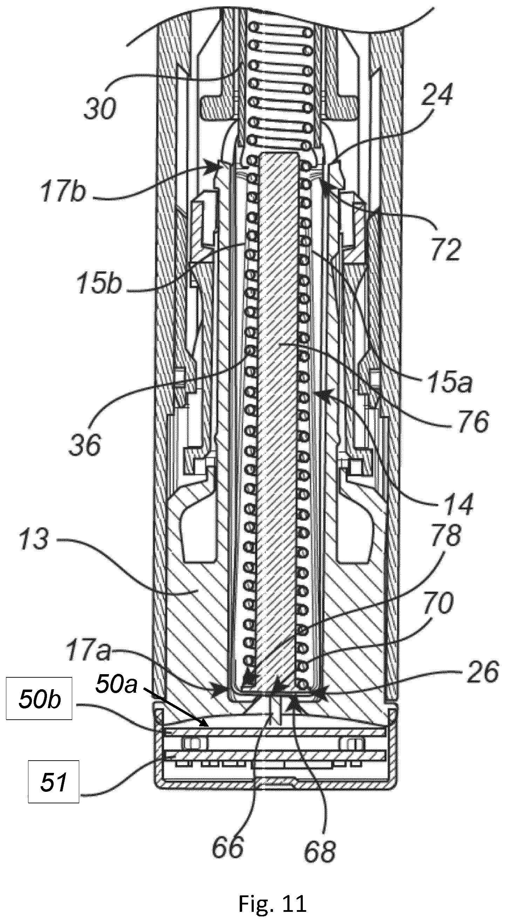

Now turning to fig. 11, where the spring 36 has pushed the plunger rod 30 further proximally to a position where the plunger rod 30 is outside the U-bracket 14. This means that the plunger rod 30 no longer provides support for maintaining the legs 15a and 15b in their original position. The distally facing surface 20 which is inclined interacts with the surface 24 of the rear cap 13 and slides thereon so that the legs 15a and 15b flexes inwards, thus, the U-bracket 14 is radially compressed whereby the distally facing surface 20 of the U-bracket is moved away from the proximally facing surface 24 of the rear cap 13. This releases the U-bracket 14 which is now movable in the distal direction under the influence of a force from the spring 36.

The motion of the U-bracket 14 in the distal direction causes the gap 28 to be closed by the U-bracket 14 that hits the proximal surface 70 of the rear cap 13, and as a consequence, the flexible portion 68 flexes in the longitudinal direction so that the pin 66 hits the first member 50a being a piezoelectric membrane which causes the first membrane 50a to move in relation to the second membrane 50b.

The piezo-electric energy harvesting unit 46 operates in ways generally known *per se* and existing piezoelectric harvesters are available. When the first piezoelectric membrane 50a is pushed and released, a voltage burst is generated. When the U-bracket bounces on the surface 70 of the rear cap multiple times, multiple voltage bursts are generated and may be buffered across e.g., a buffer capacitor within a power management unit including suitable ASICs including rectifiers and voltage conditioning components. The energy harvested from the U-bracket as it bounces on the rear cap surface 70 when the U-bracket is released is sufficient to power at least a Bluetooth low power transmitter to transmit a digital confirmation of the injection. Advantageously, a click sound is generated by the impact of the distally facing surface 42 of the U-bracket and the proximally facing surface 70 of the rear cap 13, thereby providing a direct audial confirmation of the injection to the user.

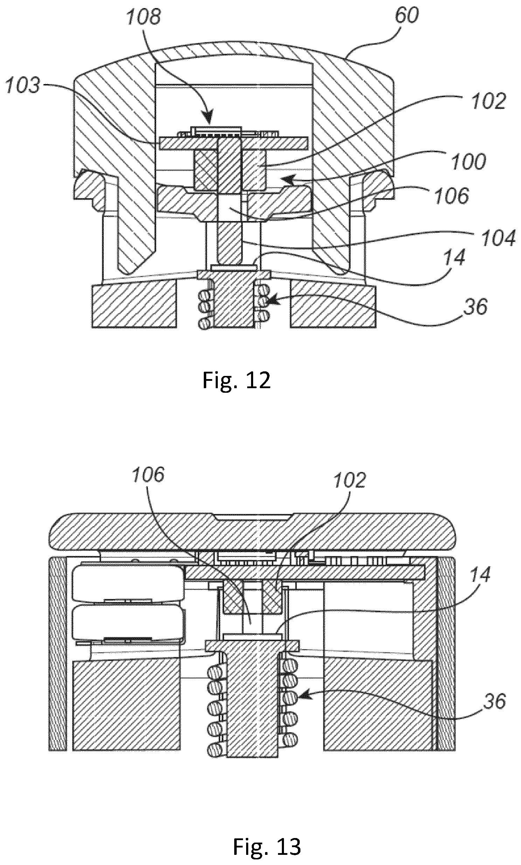

Fig. 12 illustrates a further possible implementation including an inductive harvesting unit 100 having a coil 102 arranged on a PCB 103 in the rear lid 60. Further, the inductive harvesting unit 100 includes a moving pin 104 having a magnet 106 attached thereon. As the spring is released as discussed above, the U-bracket 14 pushes on the moving pin 104 to cause the magnet 106 to move in the coil 102 which generates a voltage across the coil 102 that can be harvested by the power harvesting unit 108 on the PCB 103. In an alternative implementation, the coil 102 is movable and the pin 104 is static. The principle of inductive generation of a voltage is the same as long as a relative motion between the coil and the magnet is provided.

The coil 102 can be without a core and can allow the magnet 106 to pass through its centre. Alternatively, the coil 102 can have a metal core, where the magnet 106 will clash into the core and increase the flux, and possibly also create the end click and activate a switch to start the transmission of a digital confirmation.

Fig. 13 illustrates an alternative implementation to the disclosure of fig 12. In fig 13, the magnet 106 is attached to the U-bracket 14.

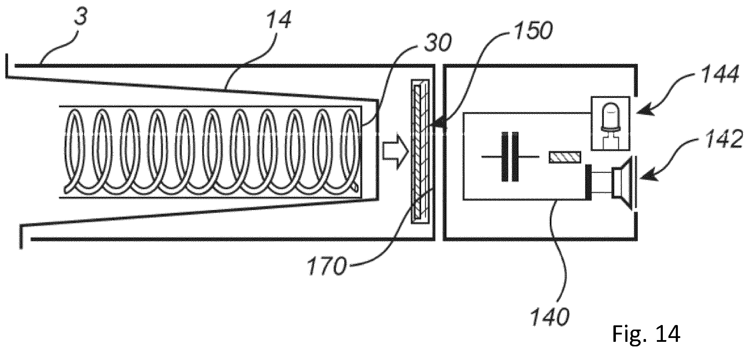

Fig. 14 conceptually illustrates yet another possible implementation. Here, a piezocrystal 150 is arranged at the point of contact between the U-bracket 14 and the proximally facing surface 170 of the housing 3. When the U-bracket 14 hits the piezocrystal 150, sufficient energy is generated and can be harvested and stored in a capacitor 140 or battery cell to be subsequently released for powering a loud speaker 142 and/or diode 144 for providing the user of an indication of when an injection is terminated and the needle can be withdrawn from the body.

A medicament delivery device (such as an autoinjector) may generally include various other components. For example, a sensor unit which may recognize injection events, such as the autoinjector being inserted into an attachment portion of e.g., a pad, injection started, and injection end, a memory unit which is configured to store the recorded data during the injection, a connectivity unit configured to transmit the stored data to a smart device or the network directly, a processing unit configured to control the entire system and to process the data before transmitting it, and/or user interface units that are configured to provide feedback to the patient, such as status LEDs, haptic, and/or audio feedback.

When the medicament delivery device is placed into the attachment portion, the sensors inside the support pad are configured to recognize the event and give feedback to the patient via haptic/visual or audio elements.

When the injection finishes, the sensors are configured to recognize the event and give feedback to the patient again. Further, the collected data is stored in the memory unit and may be transmitted to the smart device/network via the connectivity unit after the injection event finishes.

The sensor can be one of or the combination of the following: a mechanical switch, a Hall-effect sensor, an accelerometer.

The mechanical switch, hall-effect sensor, or accelerometer can be used for detection of the insertion of the auto-injector into an injection port.

The accelerometer can be used for detecting injection events.

Possible wireless communication methods include Bluetooth and Cellular Networks.

Bluetooth connectivity requires a smart device to transmit the stored data to the network and it requires a pairing action between the support pad and the smart device before being able to use the supporting pad. But it's a cheaper alternative and it requires less space on PCB.

The cellular network does not require any pairing process, it can be used as a plug-n-play device, no prior setup is needed, but it's more expensive and it requires more space on PCB.

Depending on the requirements of the product either of those two technologies can be used.

Such processing units may comprise a logic circuit or control unit including a microprocessor, microcontroller, programmable digital signal processor or another programmable device. The processing circuitry may also, or instead, each include an application specific integrated circuit, a programmable gate array or programmable array logic, a programmable logic device, or a digital signal processor. Where the processing circuitry includes a programmable device such as the microprocessor, microcontroller or programmable digital signal processor mentioned above, the processor may further include computer executable code that controls operation of the programmable device.

The medicament delivery devices described herein can be used for the treatment and/or prophylaxis of one or more of many different types of disorders. Exemplary disorders include, but are not limited to: rheumatoid arthritis, inflammatory bowel diseases (e.g. Crohn's disease and ulcerative colitis), hypercholesterolaemia, diabetes (e.g. type 2 diabetes), psoriasis, migraines, multiple sclerosis, anaemia, lupus, atopic dermatitis, asthma, nasal polyps, acute hypoglycaemia, obesity, anaphylaxis and allergies. Exemplary types of drugs that could be included in the medicament delivery devices described herein include, but are not limited to, antibodies, proteins, fusion proteins, peptibodies, polypeptides, pegylated proteins, protein fragments, protein analogues, protein variants, protein precursors, and/or protein derivatives. Exemplary drugs that could be included in the medicament delivery devices described herein include, but are not limited to (with non-limiting examples of relevant disorders in brackets): etanercept (rheumatoid arthritis, inflammatory bowel diseases (e.g. Crohn's disease and ulcerative colitis)), evolocumab (hypercholesterolaemia), exenatide (type 2 diabetes), secukinumab (psoriasis), erenumab (migraines), alirocumab (rheumatoid arthritis), methotrexate (amethopterin) (rheumatoid arthritis), tocilizumab (rheumatoid arthritis), interferon beta-1a (multiple sclerosis), sumatriptan (migraines), adalimumab (rheumatoid arthritis), darbepoetin alfa (anaemia), belimumab (lupus), peginterferon beta-1a' (multiple sclerosis), sarilumab (rheumatoid arthritis), semaglutide (type 2 diabetes, obesity), dupilumab (atopic dermatitis, asthma, nasal polyps, allergies), glucagon (acute hypoglycaemia), epinephrine (anaphylaxis), insulin (diabetes), atropine and vedolizumab (inflammatory bowel diseases (e.g. Crohn's disease and ulcerative colitis)). Pharmaceutical formulations including, but not limited to, any drug described herein are also contemplated for use in the medicament delivery devices described herein, for example pharmaceutical formulations comprising a drug as listed herein (or a pharmaceutically acceptable salt of the drug) and a pharmaceutically acceptable carrier. Pharmaceutical formulations comprising a drug as listed herein (or a pharmaceutically acceptable salt of the drug) may include one or more other active ingredients, or may be the only active ingredient present.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A subassembly (2) of a medicament delivery device (1), the subassembly comprising:
a housing (3) extending along a longitudinal axis (5) and having a proximal end (3a) and distal end (3b);
a rear cap (13) arranged inside the housing at the distal end, the rear cap comprising a flexible arm (16) that is flexible in a radial direction perpendicular to the longitudinal axis, the flexible arm comprising a protrusion (12);
a U-bracket (14) arranged radially inside the rear cap (13) and extending from a distal end (17a) of an inner space of the rear cap to a proximal end (17b) of the rear cap, the U-bracket (14) comprising at least one distally facing surface (20) on its proximal end (22) that abuts against a respective proximally facing surface (24) of the rear cap (13), and wherein a distal end (26) of the U-bracket is spaced apart from the rear cap by a gap (28);
a plunger rod (30) arranged radially inside the U-bracket, the plunger rod comprising a proximally facing surface (32) that abuts against a distally facing surface (34) of the protrusion of the rear cap;
a spring (36) arranged radially inside the plunger rod, the spring is compressed by a distally facing surface (40) of the plunger rod and a proximally facing surface (43) of the U-bracket;
a piezo-electric energy harvesting unit (46) arranged distally with respect to the rear cap (8) and comprising a first member (50a) being movable with respect to a second member (50b) for generating electrical energy, the first member (50a) being arranged adjacent the rear cap so that, when the spring (36) is released, the spring force acts on the U-bracket to close the gap (28) to cause the first member to move in relation to the second member; and
a power management unit (54) configured to harvest the electrical energy generated when the first member moves in relation to the second member and to provide the electrical energy to a data transmission device (56).

2. The subassembly according to claim 1, the piezo-electric energy harvesting unit being arranged in a rear lid (60) attached to the distal end of the housing.

3. The subassembly according to claim 2, wherein a diameter of the first member is substantially equal to an inner diameter of the lid housing the first member and the second member.

4. The subassembly according to any one of the preceding claims, wherein the rear cap comprises a pin (66) extending from the distal end of the rear cap towards the first member.

5. The subassembly according to claim 4, wherein the pin is attached to a flexible portion (68) of the rear cap.

6. The subassembly according to any one of claims 4 and 5, wherein when the spring is released, the spring force acts on the U-bracket to close the gap and impact on a surface (70) of the rear cap, whereby the pin is caused to impact on the first member.

7. The subassembly according to claim 6, wherein the U-bracket is configured to bounce on the surface of the rear cap multiple times.

8. The subassembly according to any one of the preceding claims, wherein the at least one distally facing surface (20) of the U-bracket are comprised in a respective radially extending protrusion (72).

9. The subassembly according to any one of the preceding claims, comprising a guide rod (76) arranged radially inside the spring, the guide rod comprising a flange (78) on its distal end that abuts against the distal end (80) of the spring and a distal end of the U-bracket.

10. The subassembly according to any one of the preceding claims, wherein the at least one distally facing surface (20) of the U-bracket are inclined with respect to the longitudinal axis.

11. The subassembly according to anyone of the preceding claims, wherein when the plunger rod is moved proximally in relation to the U-bracket the spring is released and decompressed, and when the plunger rod reaches to a position where the plunger rod is outside the U-bracket, the U-bracket is radially compressed so that the distally facing surface of the U-bracket is moved away from the proximally facing surface of the rear cap to be movable in the distal direction under the influence of a force from the spring.

12. The subassembly according to anyone of the preceding claims, wherein the first member (50a) is a piezo electric membrane being movable with respect to the second member (50b) being a support for the first member (50a).

13. The subassembly according to anyone of the preceding claims, wherein when the spring is released, a click sound is generated by an impact of a distally facing surface (43) of the U-bracket and a proximally facing surface (70) of the rear cap.

14. The subassembly according to anyone of the preceding claims, wherein the data transmission device includes a Bluetooth transmitter.

15. A medicament delivery device (1) comprising the subassembly according to any one of the preceding claims.

## Patentansprüche

1. Unteranordnung (2) einer Arzneimittelabgabevorrichtung (1), die Unteranordnung umfassend:
ein Gehäuse (3), das sich entlang einer Längsachse (5) erstreckt und ein proximales Ende (3a) und ein distales Ende (3b) aufweist;
eine hintere Kappe (13), die innerhalb des Gehäuses an dem distalen Ende arrangiert ist, die hintere Kappe umfassend einen flexiblen Arm (16), der in eine radiale Richtung senkrecht zu der Längsachse flexibel ist, der flexible Arm umfassend einen Vorsprung (12);
eine U-Klammer (14), die innerhalb der hinteren Kappe (13) radial arrangiert ist und sich von einem distalen Ende (17a) eines Innenraums der hinteren Kappe zu einem proximalen Ende (17b) der hinteren Kappe erstreckt, die U-Klammer (14) umfassend mindestens eine distal weisende Oberfläche (20) auf ihrem proximalen Ende (22), die gegen eine jeweilige proximal weisende Oberfläche (24) der hinteren Kappe (13) anliegt, und wobei ein distales Ende (26) der U-Klammer von der hinteren Kappe durch einen Spalt (28) beabstandet ist;
eine Kolbenstange (30), die innerhalb der U-Klammer radial arrangiert ist, die Kolbenstange umfassend eine proximal weisende Oberfläche (32), die gegen eine distal weisende Oberfläche (34) des Vorsprungs der hinteren Kappe anliegt;
eine Feder (36), die innerhalb der Kolbenstange radial arrangiert ist, wobei die Feder durch eine distal weisende Oberfläche (40) der Kolbenstange und eine proximal weisende Oberfläche (43) der U-Klammer komprimiert wird;
eine piezoelektrische Energieernteeinheit (46), die hinsichtlich der hinteren Kappe (8) distal arrangiert ist und umfassend ein erstes Element (50a), das hinsichtlich eines zweiten Elements (50b) zum Erzeugen elektrischer Energie bewegbar ist, wobei das erste Element (50a) an die hintere Kappe angrenzend arrangiert ist, sodass, wenn die Feder (36) freigegeben wird, die Federkraft auf die U-Klammer wirkt, um den Spalt (28) zu schließen, um das erste Element zu veranlassen, sich in Bezug auf das zweite Element zu bewegen; und
eine Leistungsverwaltungseinheit (54), die konfiguriert ist, um die elektrische Energie, die erzeugt wird, wenn sich das erste Element in Bezug auf das zweite Element bewegt, zu ernten und um die elektrische Energie an eine Datenübertragungsvorrichtung (56) bereitzustellen.

2. Unteranordnung nach Anspruch 1, wobei die piezoelektrische Energieernteeinheit in einem hinteren Deckel (60), der an dem distalen Ende des Gehäuses befestigt ist, arrangiert ist.

3. Unteranordnung nach Anspruch 2, wobei ein Durchmesser des ersten Elements im Wesentlichen gleich einem Innendurchmesser des Deckels ist, der das erste Element und das zweite Element aufnimmt.

4. Unteranordnung nach einem der vorstehenden Ansprüche, wobei die hintere Kappe einen Stift (66), der sich von dem distalen Ende der hinteren Kappe zu dem ersten Element hin erstreckt, umfasst.

5. Unteranordnung nach Anspruch 4, wobei der Stift an einem flexiblen Abschnitt (68) der hinteren Kappe befestigt ist.

6. Unteranordnung nach einem der Ansprüche 4 und 5, wobei, wenn die Feder freigegeben wird, die Federkraft auf die U-Klammer wirkt, um den Spalt zu schließen und auf eine Oberfläche (70) der hinteren Kappe aufzutreffen, wodurch der Stift veranlasst wird, auf das erste Element aufzutreffen.

7. Unteranordnung nach Anspruch 6, wobei die U-Klammer konfiguriert ist, um auf die Oberfläche der hinteren Kappe mehrfach zu prallen.

8. Unteranordnung nach einem der vorstehenden Ansprüche, wobei die mindestens eine distal weisende Oberfläche (20) der U-Klammer in einem jeweiligen Vorsprung (72), der sich radial erstreckt, enthalten ist.

9. Unteranordnung nach einem der vorstehenden Ansprüche, umfassend eine Führungsstange (76), die innerhalb der Feder radial arrangiert ist, die Führungsstange umfassend einen Flansch (78) auf ihrem distalen Ende, der gegen das distale Ende (80) der Feder und ein distales Ende der U-Klammer anliegt.

10. Unteranordnung nach einem der vorstehenden Ansprüche, wobei die mindestens eine distal weisende Oberfläche (20) der U-Klammer hinsichtlich der Längsachse geneigt ist.

11. Unteranordnung nach einem der vorstehenden Ansprüche, wobei, wenn die Kolbenstange in Bezug auf die U-Klammer proximal bewegt wird, die Feder freigegeben und dekomprimiert wird, und wenn die Kolbenstange eine Position erreicht, wo die Kolbenstange außerhalb der U-Klammer ist, die U-Klammer radial komprimiert wird, sodass die distal weisende Oberfläche der U-Klammer von der proximal weisenden Oberfläche der hinteren Kappe wegbewegt wird, um unter dem Einfluss einer Kraft von der Feder in die distale Richtung bewegbar zu sein.

12. Unteranordnung nach einem der vorstehenden Ansprüche, wobei das erste Element (50a) eine piezoelektrische Membran ist, die hinsichtlich des zweiten Elements (50b) bewegbar ist, wobei eine Stütze für das erste Element (50a) geboten wird.

13. Unteranordnung nach einem der vorstehenden Ansprüche, wobei, wenn die Feder freigegeben wird, ein Klickgeräusch durch ein Auftreffen einer distal weisenden Oberfläche (43) der U-Klammer und einer proximal weisenden Oberfläche (70) der hinteren Kappe erzeugt wird.

14. Unteranordnung nach einem der vorstehenden Ansprüche, wobei die Datenübertragungsvorrichtung einen Bluetooth-Sender einschließt.

15. Arzneimittelabgabevorrichtung (1), umfassend die Unteranordnung nach einem der vorstehenden Ansprüche.

## Revendications

1. Sous-ensemble (2) d'un dispositif d'administration de médicament (1), le sous-ensemble comprenant :
un boîtier (3) s'étendant le long d'un axe longitudinal (5) et ayant une extrémité proximale (3a) et une extrémité distale (3b) ;
un capuchon arrière (13) agencé à l'intérieur du boîtier au niveau de l'extrémité distale, le capuchon arrière comprenant un bras flexible (16) qui est flexible dans une direction radiale perpendiculaire à l'axe longitudinal, le bras flexible comprenant une protubérance (12) ;
un support en U (14) disposé radialement à l'intérieur du capuchon arrière (13) et s'étendant d'une extrémité distale (17a) d'un espace interne du capuchon arrière à une extrémité proximale (17b) du capuchon arrière, le support en U (14) comprenant au moins une surface orientée distalement (20) sur son extrémité proximale (22) qui vient en butée contre une surface orientée proximalement (24) respective du capuchon arrière (13), et dans lequel une extrémité distale (26) du support en U est espacée du capuchon arrière par un espace (28) ;
une tige de piston (30) agencée radialement à l'intérieur du support en U, la tige de piston comprenant une surface orientée proximalement (32) qui vient en butée contre une surface orientée distalement (34) de la protubérance du capuchon arrière ;
un ressort (36) agencé radialement à l'intérieur de la tige de piston, le ressort étant comprimé par une surface orientée distalement (40) de la tige de piston et une surface orientée proximalement (43) du support en U ;
une unité de récupération d'énergie piézo-électrique (46) agencée distalement par rapport au capuchon arrière (8) et comprenant un premier élément (50a) mobile par rapport à un second élément (50b) pour produire de l'énergie électrique, le premier élément (50a) étant agencé adjacent au capuchon arrière de sorte que, lorsque le ressort (36) est relâché, la force de ressort agit sur le support en U pour fermer l'espace (28) afin de provoquer le déplacement du premier élément par rapport au second élément ; et
une unité de gestion d'alimentation (54) configurée pour récupérer l'énergie électrique produite lorsque le premier élément se déplace par rapport au second élément et pour fournir l'énergie électrique à un dispositif de transmission de données (56).

2. Sous-ensemble selon la revendication 1, l'unité de récupération d'énergie piézo-électrique étant agencée dans un couvercle arrière (60) fixé à l'extrémité distale du boîtier.

3. Sous-ensemble selon la revendication 2, dans lequel un diamètre du premier élément est sensiblement égal à un diamètre interne du couvercle logeant le premier élément et le second élément.

4. Sous-ensemble selon l'une quelconque des revendications précédentes, dans lequel le capuchon arrière comprend une goupille (66) s'étendant de l'extrémité distale du capuchon arrière vers le premier élément.

5. Sous-ensemble selon la revendication 4, dans lequel la goupille est fixée à une partie flexible (68) du capuchon arrière.

6. Sous-ensemble selon l'une quelconque des revendications 4 et 5, dans lequel, lorsque le ressort est relâché, la force de ressort agit sur le support en U pour fermer l'espace et frapper une surface (70) du capuchon arrière, de telle manière que la goupille est amenée à frapper sur le premier élément.

7. Sous-ensemble selon la revendication 6, dans lequel le support en U est conçu pour rebondir plusieurs fois sur la surface du capuchon arrière.

8. Sous-ensemble selon l'une quelconque des revendications précédentes, dans lequel l'au moins une surface orientée distalement (20) du support en U est comprise dans une protubérance s'étendant radialement (72) respective.

9. Sous-ensemble selon l'une quelconque des revendications précédentes, comprenant une tige de guidage (76) agencée radialement à l'intérieur du ressort, la tige de guidage comprenant une bride (78) sur son extrémité distale qui vient en butée contre l'extrémité distale (80) du ressort et une extrémité distale du support en U.

10. Sous-ensemble selon l'une quelconque des revendications précédentes, dans lequel l'au moins une surface orientée distalement (20) du support en U est inclinée par rapport à l'axe longitudinal.

11. Sous-ensemble selon l'une quelconque des revendications précédentes, dans lequel lorsque la tige de piston est déplacée proximalement par rapport au support en U, le ressort est relâché et décompressé, et lorsque la tige de piston atteint une position où la tige de piston est à l'extérieur du support en U, le support en U est comprimé radialement de sorte que la surface orientée distalement du support en U est éloignée de la surface orientée proximalement du capuchon arrière pour pouvoir être déplacée à l'intérieur la direction distale sous l'influence d'une force provenant du ressort.

12. Sous-ensemble selon l'une quelconque des revendications précédentes, dans lequel le premier élément (50a) est une membrane piézo-électrique mobile par rapport au second élément (50b) qui est un support pour le premier élément (50a).

13. Sous-ensemble selon l'une quelconque des revendications précédentes, dans lequel lorsque le ressort est relâché, un cliquetis est généré par un impact d'une surface orientée distalement (43) du support en U et d'une surface orientée proximalement (70) du capuchon arrière.

14. Sous-ensemble selon l'une quelconque des revendications précédentes, dans lequel le dispositif de transmission de données comporte un émetteur Bluetooth.

15. Dispositif d'administration de médicament (1) comprenant le sous-ensemble selon l'une quelconque des revendications précédentes.
